(19) Europäisches Patentamt
European Patent Office
Office européen des brevets

(11) Veröffentlichungsnummer: **0 293 711 B1**

# (12) EUROPÄISCHE PATENTSCHRIFT

(45) Veröffentlichungstag der Patentschrift: **11.12.91**

(51) Int. Cl.$^5$: **C07D 207/34**, C07D 207/22

(21) Anmeldenummer: **88108229.1**

(22) Anmeldetag: **24.05.88**

## (54) Verfahren zur Herstellung von 3-Cyano-4-aryl-pyrrolen.

(30) Priorität: **02.06.87 DE 3718375**

(43) Veröffentlichungstag der Anmeldung:
**07.12.88 Patentblatt 88/49**

(45) Bekanntmachung des Hinweises auf die Patenterteilung:
**11.12.91 Patentblatt 91/50**

(84) Benannte Vertragsstaaten:
**BE CH DE FR GB IT LI NL**

(56) Entgegenhaltungen:
**EP-A- 0 174 910**
**EP-A- 0 182 738**
**EP-A- 0 183 217**
**DE-A- 2 927 480**

**HOUBEN-WEYL - Methoden der Organischen Chemie, Band E5, 1985, Seiten1654-1657**

(73) Patentinhaber: **BAYER AG**
**W-5090 Leverkusen 1 Bayerwerk(DE)**

(72) Erfinder: **Wollweber, Detlef, Dr.**
**Paul-Ehrlich-Strasse 17**
**W-5600 Wuppertal 1(DE)**

EP 0 293 711 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft ein neues Verfahren zur Herstellung von 3-Cyano-4-aryl-pyrrolen, welche als Fungizide bekannt sind, sowie neue Zwischenprodukte zur Herstellung derselben.

Es ist bekannt, daß man 3-Cyano-4-aryl-pyrrole erhält, wenn man Zimtsäurenitrile in Gegenwart von Natriumhydrid mit p-Toluolsulfonylmethylisocyanid umsetzt (vgl. DE-OS 2 927 480). Dieses Verfahren bringt jedoch mit ca 35 % Ausbeute nur unbefriedigende Ergebnisse. Nachteilig ist außerdem, daß die so erhältlichen Verbindungen aufwendig gereinigt werden müssen (vgl. J6-1030-571). Schließlich sind die Reagenzien Natriumhydrid und p-Toluolsulfonylmethylisocyanid beide für technische Synthesen ungeeignet, ersteres wegen der hohen Hydrolyseanfälligkeit und der damit verbundenen Brandgefahr des bei der Hydrolyse freigesetzten gasförmigen Wasserstoffes, zweiteres wegen der starken Reizwirkung auf Haut und Augen und der leichten Zersetzlichkeit bei erhöhter Temperatur (vgl. EP 174 910).

Weiterhin ist bekannt, daß man 3-Cyano-4-aryl-pyrrole auch erhält, wenn man α-Cyanozimtsäureester in Gegenwart von Basen und in Gegenwart von Kupfer-(II)-Salzen mit p-Toluolsulfonylmethylisocyanid umsetzt (vgl. J6-1030-571 oder J6-1200-984). Auch bei diesem Verfahren stehen die Eigenschaften des p-Toluolsulfonylmethylisocyanids einer technischen Anwendung im Wege.

Außerdem ist bekannt, daß man 3-Cyano-4-aryl-pyrrole auch erhält, wenn man α-substituierte Zimtsäurenitrile in Gegenwart von Natriumhydrid mit Isocyanoessigsäureethylester cyclisiert, die so erhältlichen Pyrrol-2-carbonsäureester mit Basen verseift und anschließend thermisch decarboxyliert (vgl. JP 59/212 468). Der technischen Anwendbarkeit dieses Verfahrens stehen wiederum die ungünstigen Eigenschaften des Natriumhydrids im Wege. Auch die Ausbeuten des Cyclisierungsschrittes sind mit 44 % nicht zufriedenstellend.

Weiterhin ist bekannt, daß man 3-Cyano-4-aryl-pyrrole erhält, wenn man Phenacylamin-Derivate mit geeignet substituierten Acrylnitril-Derivaten umsetzt (vgl. EP 174 910). Die aus Ausgangsverbindungen erforderlichen Phenacylaminderivate sind jedoch nur über eine aufwendige, vielstufige Synthese zugänglich, in deren Verlauf unter anderem auch der unerfreuliche Einsatz von Cyaniden notwendig ist.

Weiterhin ist bekannt, daß man 3-Cyano-4-aryl-pyrrole erhält, wenn man die entsprechenden 3-Trifluormethyl-4-aryl-pyrrole mit Ammoniak bei erhöhter Temperatur und erhöhtem Druck umsetzt (vgl. EP 182 738). Auch bei diesem Verfahren sind jedoch die als Ausgangsprodukte erforderlichen 3-Trifluormethyl-4-aryl-pyrrole nur über einen aufwendigen, mehrstufigen Weg zugänglich, wobei die Verwendung von feuchtigkeitsempfindlichen "Wittig-Reagenzien" und kostspieligem Trifluoracetanhydrid im Verlauf dieser mehrstufigen Synthese die technische Durchführbarkeit zusätzlich erschwert.

Schließlich ist bekannt, daß man 3-Cyano-4-aryl-pyrrole erhält, wenn man 4-Cyano-3-aryl-$\Delta^2$-pyrroline in Gegenwart von Cu-II-Salzen oder Eisen-III-salzen oxidiert (vgl. EP 183 217). Auch bei diesem letzten Verfahren ist die Herstellung der erforderlichen Ausgangsverbindungen mehrstufig und technisch aufwendig.

Es wurde gefunden, daß man 3-Cyano-4-aryl-pyrrole der allgemeinen Formel (I),

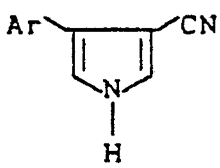

(I)

in welcher

Ar für jeweils gegebenenfalls substituiertes Heteroaryl oder Aryl steht,

erhält, wenn man α-Cyanoacrylsäurederivate der Formel (II),

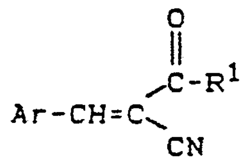

(II)

in welcher

Ar die oben angegebene Bedeutung hat und
$R^1$ für Amino oder Alkoxy steht, mit Isocyanessigsäureestern der Formel (III),

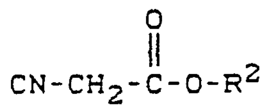 (III)

in welcher
$R^2$ für Alkyl steht,
in Gegenwart einer Base und gegebenenfalls in Gegenwart eines Verdünnungsmittels umsetzt und die so erhältlichen $\Delta^2$-Pyrrolin-2-carbonsäurederivate der Formel (IVa),

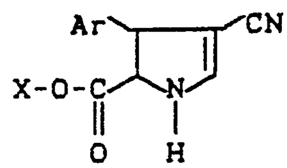 (IVa)

in welcher
X für Wasserstoff oder für ein Äquivalent eines anorganischen oder organischen Kations steht und
Ar die oben angegebene Bedeutung hat,
in einer 2. Stufe gegebenenfalls in Gegenwart einer Base und in Gegenwart eines Metallsalzes sowie gegebenenfalls in Gegenwart eines Verdünnungsmittel oxidativ decarboxyliert.

Es ist als ausgesprochen überraschend anzusehen, daß die Cyclisierung von α-Cyanoacrylsäurederivaten der Formel (II) mit Isocyanoessigestern der Formel (III) zu $\Delta^2$-Pyrrolin-2-carbonsäurederivaten führt, da aufgrund des Standes der Technik zu erwarten war, daß unter den gegebenen Reaktionsbedingungen bei der Cyclisierung der Verbindungen bevorzugt Cyanwasserstoff abgespalten würde und daher eher Pyrrol-Derivate als Pyrrolin-Derivate resultieren sollten (vgl. JP 59/ 212 468). Darüberhinaus ist es auch völlig überraschend, daß die in der 2. Stufe des erfindungsgemäßen Verfahrens durchgeführte oxidative Decarboxylierung der $\Delta^2$-Pyrrolin-2-carbonsäure-Derivate unter derart milden Bedingungen in Gegenwart eines Kupfer-II-Salz-Katalysators abläuft, da aus dem Stand der Technik bekannt war, daß Kupfer-II-Salze alleine unter milden Reaktionsbedingungen keine oxidative Decarboxylierung bewirken können (vgl. Organic Reactions Vol. 19, S. 279, 303 ff).

Ein besonderer Vorteil des erfindungsgemäßen Verfahrens besteht in der Verwendung von leicht zugänglichen preiswerten Ausgangsstoffen; darüberhinaus stellt die Tatsache, daß man ohne aufwendige Reinigungsoperationen Produkte hoher Reinheit in guten Ausbeuten erhält, einen weiteren Vorteil des erfindungsgemäßen Verfahrens dar.

Die mit Hilfe des erfindungsgemäßen Verfahrens erhältlichen 3-Cyano-4-aryl-pyrrole sind durch die Formel (I) allgemein definiert.

Bevorzugt herstellbar sind Verbindungen der Formel (I), bei welchen
Ar für jeweils gegebenenfalls einfach bis mehrfach, gleich oder verschieden durch Halogen und geradkettiges oder verzweigtes Alkyl mit 1 bis 4 Kohlenstoffatomen substituiertes Pyridyl, Furyl oder Thienyl steht oder für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen sowie zweifach verknüpftes, gegebenenfalls durch Fluor substituiertes Dioxyalkylen mit 1 oder 2 Kohlenstoffatomen.

Besonders bevorzugt herstellbar sind Verbindungen der Formel (I), bei welchen
Ar für jeweils gegebenenfalls einfach oder zweifach, gleich oder verschieden durch Fluor, Chlor, Brom, Methyl und Ethyl substituiertes 2-Pyridyl, 4-Pyridyl, 2-Furyl oder 2-Thienyl steht oder für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, Methoxy,

Ethoxy, Methylthio, Trifluormethyl, Trifluormethoxy, Trifluormethylthio, Cyano, Nitro, Dioxymethylen und Dioxydifluormethylen.

Ganz besonders bevorzugt herstellbar sind Verbindungen der Formel (I), bei welchen

Ar für gegebenenfalls einfach oder zweifach, gleich oder verschieden substituiertes Phenyl steht, wobei als Substituenten infrage kommen: Fluor, Chlor, Brom, Methyl, Ethyl, Methoxy, Ethoxy, Methylthio, Trifluormethyl, Trifluormethoxy, Trifluormethylthio, Nitro und Dioxydifluormethylen.

Verwendet man beispielsweise 2-(2,3-Dichlorphenylmethyliden)-cyanessigsäureethylester und Isocyanoessigsäureethylester als Ausgangsstoffe, so läßt sich der Reaktionsablauf des erfindungsgemäßen Verfahrens durch das folgende Formelschema darstellen:

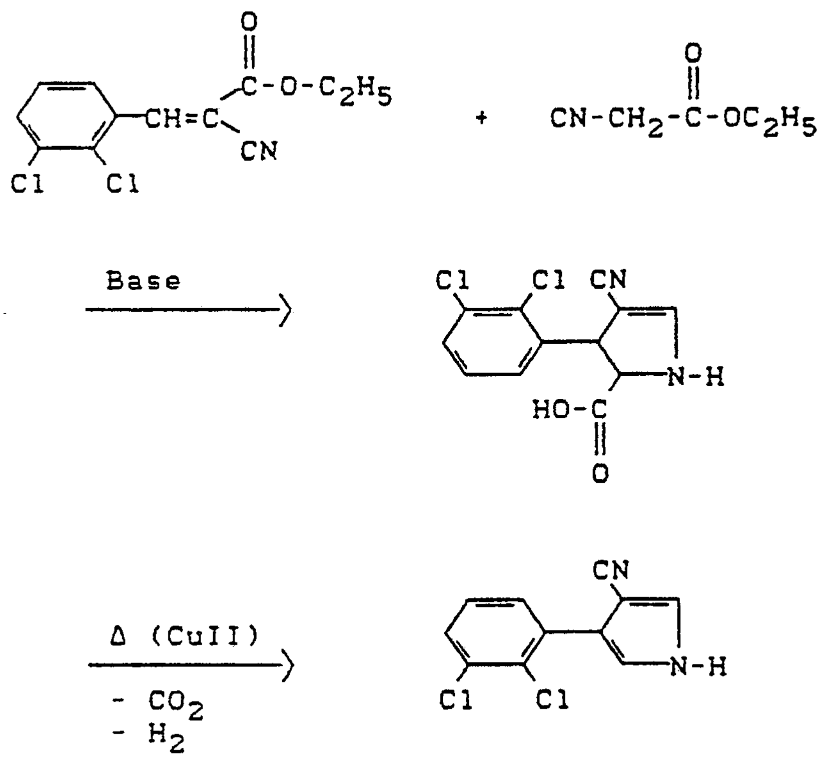


Die zur Durchführung des erfindungsgemäßen Verfahrens als Ausgangsstoffe benötigten α-Cyanoacrylsäurederivate sind durch die Formel (II) allgemein definiert. In dieser Formel (II) steht Ar vorzugsweise für diejenigen Reste, die bereits im Zusammenhang mit der Beschreibung der erfindungsgemäß herstellbaren Stoffe der Formel (I) als bevorzugt für diesen Substituenten genannt wurden.

$R^1$ steht vorzugsweise für geradkettiges oder verzweigtes Alkoxy mit 1 bis 4 Kohlenstoffatomen, insbesondere für Methoxy and Ethoxy oder für Amino.

Die α-Cyanacrylsäurederivate der Formel (II) sind bekannt (vgl. z.B. J6-1030-571 oder J6-1200-984) oder können in Analogie zu bekannten Verfahren hergestellt werden (vgl. auch J. Chem. Soc. 1961, 683), beispielsweise wenn man Aldehyde der Formel (V),

Ar-CHO (V)

in welcher

Ar die oben angegebene Bedeutung hat,

mit Cyanessigsäurederivaten der Formel (VI),

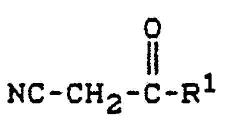
(VI)

in welcher

$R^1$ die oben angegebene Bedeutung hat,

gegebenenfalls in Gegenwart eines Verdünnungsmittels wie beispielsweise Ethanol und gegebenenfalls in Gegenwart einer Base wie beispielsweise Kaliumhydroxid oder Piperidin bei Temperaturen zwischen + 20 °C und + 150 °C kondensiert.

Die Aldehyde der Formel (V) und die Cyanessigsäurederivate der Formel (VI) sind allgemein bekannte Verbindungen der organischen Chemie.

Die zur Durchführung des erfindungsgemäßen Verfahrens weiterhin als Ausgangsstoffe benötigten Isocyanessigsäureester sind durch die Formel (III) allgemein definiert. In dieser Formel (III) steht $R^2$ vorzugsweise für geradkettiges oder verzweigtes Alkyl mit 1 bis 4 Kohlenstoffatomen, insbesondere für Methyl oder Ethyl.

Die Isocyanessigsäureester sind bekannt (vgl. z.B. JP 59/212 sowie Liebigs Ann. Chem. 763, 1, [1972]).

Als Verdünnungsmittel zur Durchführung der 1. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Alkohole, wie Methanol oder Ethanol.

Die 1. Stufe des erfindungsgemäßen Verfahrens wird vorzugsweise in Gegenwart einer geeigneten Base durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU). Mit besonderem Vorzug verwendet man Kaliumhydroxid als Base.

Die Reaktionstemperaturen können bei der Durchführung der 1. Stufe des erfindungsgemäßen Verfahrens in einem größeren Bereich variiert werden. Im allgemeinen arbeitet man bei Temperaturen zwischen -20 °C und + 100 °C, vorzugsweise bei Temperaturen zwischen 0 °C und 50 °C.

Zur Durchführung der 1. Stufe des erfindungsgemäßen Verfahrens setzt man pro Mol an α-Cyanacrylsäurederivat der Formel (II) im allgemeinen 1.0 bis 2,0 Mol, vorzugsweise 1.0 bis 1,2 Mol an Isocyanessigsäureester der Formel (III) und gegebenenfalls 1.0 bis 6,0 Mol, vorzugsweise 2.0 bis 3,0 Mol an Base ein. Die Reaktionsdurchführung, Aufarbeitung und Isolierung der Reaktionsprodukte erfolgt nach allgemein üblichen Methoden (vgl. auch die Herstellungsbeispiele).

Führt man die 1.Stufe des erfindungsgemäßen Verfahrens bei niedriger Temperatur (-20 °C bis + 30 °C) in einem aprotischen Verdünnungsmittel wie beispielsweise Tetrahydrofuran oder Dimethylformamid durch und verwendet nur geringe molare Überschüsse an Hydroxid-Base oder wasserfreie organische Basen wie beispielsweise Kalium-t-butylat, so lassen sich die als Zwischenprodukte bei der Reaktion auftretenden $\Delta^2$-Pyrrolin-2-carbonsäureester der Formel (IVb),

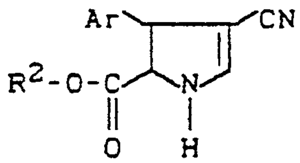

(IVb)

in welcher

$R^2$ für Alkyl, insbesondere für Methyl oder Ethyl steht und

Ar die oben angegebene Bedeutung hat,

isolieren.

Sie können dann in einem separaten Reaktionsschritt in üblicher Art und Weise verseift werden zu den Zwischenprodukten der Formel (IVa).

Bevorzugt ist jedoch bei der 1. Stufe des erfindungsgemäßen Verfahrens eine Reaktionsführung, bei der die Zwischenprodukte der Formel (IVb) nicht isoliert werden.

Als Verdünnungsmittel zur Durchführung der 2. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Alkohole, wie Methanol oder Ethanol, oder deren Gemische mit Wasser.

Die 2. Stufe des erfindungsgemäßen Verfahrens wird vorzugsweise in Gegenwart eines geeigneten Schwermetallsalzes durchgeführt. Mit besonderem Vorzug verwendet man Cu-I- oder Cu-II-Salze wie Kupferacetat oder Kupferchlorid oder Eisen-III-Salze wie beispielsweise Eisen-III-chlorid.

Die 2. Stufe des erfindungsgemäßen Verfahrens wird vorzugsweise in Gegenwart einer geeigneten Base durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 2. Stufe des erfindungsgemäßen Verfahrens in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung der 2. Stufe des erfindungsgemäßen Verfahrens setzt man pro Mol an $\Delta^2$-Pyrrolin-2-carbonsäurederivat der Formel (IVa) im allgemeinen 0,01 bis 3,0 Mol, vorzugsweise 0.1 bis 0.5 Mol an Metallsalz und 0,1 bis 3,0 Mol, vorzugsweise 1.0 bis 1,5 Mol an Base ein.

Setzt man das Metallsalz nur in katalytischen Mengen ein, so ist es von Vorteil zusätzlich Luft oder reinen Sauerstoff in die Reaktionsmischung einzuleiten um reduzierte Metallionen wieder aufzuoxidieren. Die Reaktionsdurchführung, Aufarbeitung und Isolierung der Reaktionsprodukte erfolgt nach allgemein üblichen Methoden (vgl. auch die Herstellungsbeispiele).

Die mit Hilfe des erfindungsgemäßen Verfahrens erhältichen Verbindungen der Formel (I) sind bekannte Verbindungen, welche als Fungizide oder Mikrobizide und als Zwischenprodukte zur Synthese weiterer Fungizide oder Mikrobizide Verwendung finden (vgl. z.B. EP 96 142, EP 111 452; DE-OS 2 927 480).

Die Zwischenprodukte der Formel (IV),

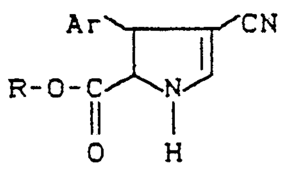

(IV)

in welcher

R für Wasserstoff, Alkyl oder für ein Äquivalent eines anorganischen oder organischen Kations steht; vorzugsweise für Wasserstoff, geradkettiges oder verzweigtes Alkyl mit 1 bis 4 Kohlenstoffatomen oder für 1 Äquivalent eines Alkalimetall-, Erdalkalimetall- oder gegebenenfalls substituierten Ammoniumkations steht; insbesondere für Wasserstoff, Methyl, Ethyl oder für ein Äquivalent eines Natrium-, Kalium- oder Ammonium- sowie Mono-, Di-oder Trialkylammoniumions (mit jeweils 1 bis 4 Kohlenstoffatomen in den einzelnen Alkylteilen) steht und

Ar die oben angegebene Bedeutung hat,

sind neu und ebenfalls Gegenstand der vorliegenden Erfindung. Sie besitzen außer ihren wertvollen Eigenschaften als Zwischenprodukte zur Synthese von fungiziden oder mikrobiziden Wirkstoffen auch selbst fungizide sowie mikrobizide Eigenschaften.

Herstellungsbeispiele

Beispiel 1

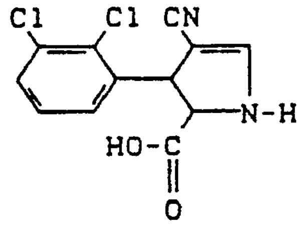

(IVa-1)

(1. Stufe)

Zu 2,4 g (0,04 Mol) Kaliumhydroxid in 100 ml Ethanol gibt man bei -5 °C bis 0 °C eine Suspension aus 4,8 g (0,02 Mol) 2-Cyano-3-(2,3-dichlorphenyl)-acrylsäureamid in 50 ml Ethanol, tropft anschließend 2,6 g (0,022 Mol) Isocyanessigsäureethylester zu und rührt nach beendeter Zugabe 4 Stunden bei Raumtemperatur. Zur Aufarbeitung gibt man 200 ml Wasser zu, extrahiert mit Essigester, verwirft die organische Phase, säuert die wässrige Phase mit 1 normaler Chlorwasserstoffsäure an und extrahiert ein zweites Mal mit Essigester. Aus den vereinigten Essigesterphasen der 2. Extraktion erhält man durch Zugabe von Petrolether einen Feststoff, der nach Abfiltrieren und Trocknen einen Schmelzpunkt von 200 °C bis 202 °C besitzt und laut

Hochdruckflüssigchromatogramm eine Reinheit von 95 % besitzt.

$^1$H-NMR (DMSO-$d_6$/TMS):

$\delta$ = 4,3 (d, 1H); 4,8 (d, 1H); 7,3 (d, 1H); 7,4 (t, 1H); 7,5 (s, 1H); 7,6 (m, 2H); 13,0-13,5 (m, 1H) ppm.

$^{13}$C-NMR (DMSO-$d_6$):

$\delta$ = 48,3; 67,1; 77,9; 118,9; 127,7; 129,0; 129,8; 130,6; 132,3; 141,7; 152,7; 172,4 ppm.

MS : m/e = 282 ($M^+$)

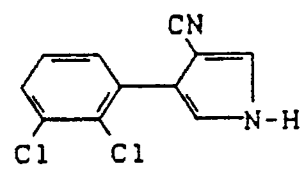

(I-1)

(2. Stufe)

Zu einer Mischung an 2 g (0,01 Mol) Kupferacetat-monohydrat und 2 ml (0,025 Mol) Pyridin in 100 ml eines Toluol/Essigester-Gemisches (1:1) gibt man unter Rühren bei Raumtemperatur 5,6 g (0,02 Mol) 3-(2,3-Dichlorphenyl)-4-cyano-$\Delta^2$-pyrrolin-2-carbonsäure und erhitzt anschließend für 6 Stunden auf Rückflußtemperatur. Nach Abkühlen der Reaktionsmischung filtriert man unlösliche Bestandteile ab, wäscht nacheinander mit 1 normaler Salzsäure, wässriger Natriumcarbonatlösung und Wasser, trocknet über Natriumsulfat und engt im Vakuum ein. Nach Digerieren des Rückstandes mit Diisopropylether erhält man das 3-Cyano-4-(2,3-dichlorphenyl)-pyrrol als Feststoff vom Schmelzpunkt 149 °C - 150 °C mit einer gaschromatographisch bestimmten Reinheit von 98 %.

$^1$H-NMR (DMSO-$d_6$/TMS):

$\delta$ = 7,2 (d, 1H); 7,4 - 7,5 (m, 2H) 7,65 (m, 1H); 7,75 (d, 1H) ppm.

<u>Herstellung der Zwischenprodukte (IVb):</u>

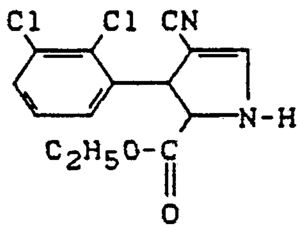

(IVb-1)

Zu einer Suspension aus 2,4 g (0,021 Mol) Kalium-t-butylat in 50 ml Tetrahydrofuran tropft man unter Rühren bei 0 °C bis 10 °C eine Lösung aus 4,8 g (0,02 Mol) 2-Cyano-3-(2,3-dichlorphenyl)-acrylsäureamid und 2,5 g (0,022 Mol) Isocyanessigsäureethylester in 60 ml einer Tetrahydrofuran/Dimethylformamid-Mischung (5:1). Nach beendeter Zugabe rührt man weitere 5 Stunden bei Raumtemperatur, gießt dann die Reaktionsmischung in 250 ml Wasser, extrahiert dreimal mit Diethylether, wäscht die vereinigten Etherphasen mit Wasser, trocknet über Natriumsulfat und engt im Vakuum ein. Der ölige Rückstand wird durch Chromatographie an Kieselgel (Laufmittel: Essigester/Cyclohexan) und Digerieren mit Diisopropylether gereinigt. Der so erhältiche 3-Cyano-4-(2,3-dichlorphenyl)-$\Delta^2$-pyrrolin-5-carbonsäureethylester hat einen Schmelzpunkt von 124 °C - 125 °C.

$^1$H-NMR ($CDCl_3$/TMS):

$\delta$ = 1,3 (t, 3H); 4,2 (d, 1H); 4,3 (m, 2H); 5,0 (d, 1H); 7,1 - 7,3 (m, 4H); 7,4 (m, 1H) ppm.

MS: m/e = 310 ($M^+$)

Die folgenden Produkte können in analoger Weise wie oben und in Übereinstimmung mit den allgemeinen Angaben zur Herstellung hergestellt werden:

Beispiel IVa-2

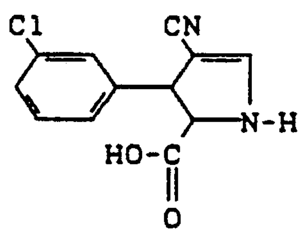

Schmelzpunkt 180° C - 181° C

Beispiel IVb-2

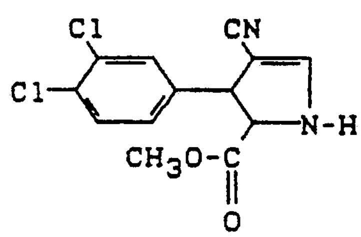

Schmelzpunkt 154° C - 156° C

**Patentansprüche**

1. Verfahren zur Herstellung von 3-Cyano-4-aryl-pyrrolen der allgemeinen Formel (I),

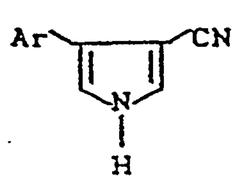

(I)

in welcher

Ar für jeweils gegebenenfalls einfach bis mehrfach, gleich oder verschieden durch Halogen und geradkettiges oder verzweigtes Alkyl mit 1 bis 4 Kohlenstoffatomen substituiertes Pyridyl, Furyl oder Thienyl steht oder für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio oder Alkoxycarbonyl mit jeweils 1 - 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen sowie zweifach verknüpftes,gegebenenfalls durch Fluor substituiertes Dioxyalkylen mit 1 oder 2 Kohlenstoffatomen steht,

dadurch gekennzeichnet, daß man α-Cyanoacrylsäurederivate der Formel (II),

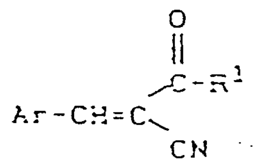

(II)

in welcher

Ar die oben angegebene Bedeutung hat und
$R^1$ für Amino oder Alkoxy mit 1 bis 4 Kohlenstoffatomen steht,

mit Isocyanessigsäureestern der Formel (III),

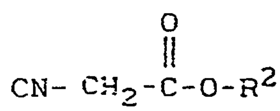

(III)

in welcher

$R^2$ für Alkyl mit 1 bis 4 Kohlenstoffatomen steht,
in Gegenwart von Natrimhydrid, Natriumamid, Natriumhydroxid oder Kaliumhydroxid und gegebenenfalls in Gegenwart eines Verdünnungsmittels bei Temperaturen zwischen 0 - 50 °C umsetzt, wobei man pro Mol an α-Cyanacrylsäurederivat der Formel (II) 1.0 bis 2.0 Mol an Isocyanessigsäureester der Formel (III) und 1.0 bis 6.0 Mol der oben angegebenen Basen einsetzt, und die so erhältlichen $\Delta^2$-Pyrrolin-2-carbonsäurederivate der Formel (IVa),

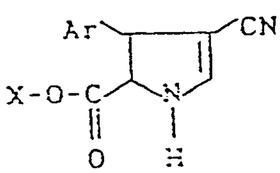

(IVa)

in welcher

X für Wasserstoff oder für ein Äquivalent eines anorganischen oder organischen Kations steht und
Ar die oben angegebene Bedeutung hat,

in einer 2. Stufe gegebenenfalls in Gegenwart eines Alkalihydroxids, Alkalimetallcarbonats oder eines

tertiären Amins und in Gegenwart eines Kupfer- oder Eisen(III)-salzes sowie gegebenenfalls in Gegenwart eines Verdünnungsmittels bei Temperaturen zwischen 20 und 120 °C oxidativ decarboxyliert, wobei man pro Mol $\Delta^2$-Pyrrolin-2-carbonsäure-derivat der Formel (IVa) 0,01 bis 3,0 Mol an Kupfer- oder Eisen(III)-salz und gegebenenfalls 0,1 bis 3,0 Mol an einer der oben aufgeführten Basen einsetzt.

2. Verfahren nach Anspruch 1, dadurch gekennzeichnet, daß man die 1. und 2. Stufe ohne Isolierung der Zwischenprodukte der Formel (IVb)

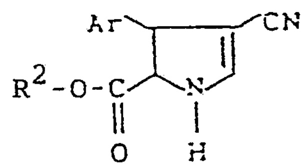

(IV b)

in welcher

$R^2$ und Ar die in Anspruch 1 gegebenen Bedeutungen haben, durchführt.

3. Verfahren nach Anspruch 1, dadurch gekennzeichnet, daß man die 1. und 2. Stufe in Gegenwart von Organischen Lösungsmitteln durchführt.

4. Verfahren nach Anpruch 1, dadurch gekennzeichnet, daß man die 1. und 2. Stufe in Gegenwart der in Anspruch 1 aufgeführten Basen durchführt.

5. Verfahren nach Anspruch 1, dadurch gekennzeichnet, daß man die 2. Stufe in Gegenwart eines Kupfer- oder Eisen(III)-salzes durchführt.

6. Pyrrol-2-carbonsäurederivate der allgemeinen Formel (IV), gemäß Anspruch 1

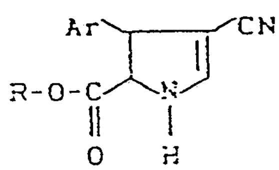

(IV)

in welcher

R für Wasserstoff, Alkyl mit 1 bis 4 Kohlenstoffatomen oder für ein Äquivalent eines anorganischen oder organischen Kations steht und Ar die in Anspruch 1 angegebene Bedeutung hat.

7. Verfahren zur Herstellung von $\Delta^2$-Pyrrolin-2-carbonsäurederivatender allgemeinen Formel (IV) gemäß Anspruch 1

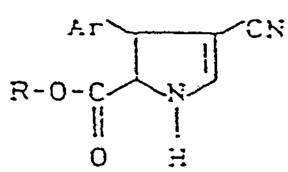

(IV)

in welcher

R für Wasserstoff, Alkyl mit 1 bis 4 Kohlenstoffatomen oder für ein Äquivalent eines anorganischen oder organischen Kations steht und Ar die in Anspruch 1 angegebene Bedeutung hat,

dadurch gekennzeichnet, daß man α-Cyanacrylsäurederivate der Formel (II),

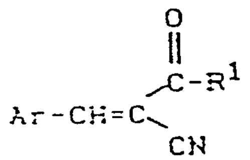

(II)

in welcher

Ar die oben angegebene Bedeutung hat und
$R^1$ für Amino oder Alkoxy mit 1 bis 4 Kohlenstoffatomen steht,

mit Isocyanessigsäureestern der Formel (III),

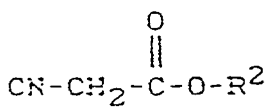

(III)

in welcher

$R^2$ für Alkyl mit 1 bis 4 Kohlenstoffatomen steht,

in einem aprotischen Lösungsmittel bei Temperaturen zwischen - 20 °C und 30 °C in Gegenwart quasi molarer Mengen an Hydroxyd-Base oder wasserfreien organischen Basen umsetzt und gegebenenfalls die erhaltenen Verbindungen verseift.

**Claims**

1. Process for the preparation of 3-cyano-4-aryl-pyrroles of the general formula (I)

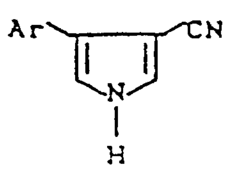

(I)

in which
Ar represents pyridyl, furyl or thienyl which are optionally monosubstituted to polysubstituted in each case by identical or different substituents from the group comprising halogen and straight-chain or branched alkyl having 1 to 4 carbon atoms or represents phenyl which is optionally monosubstituted to polysubstituted by identical or different substituents, suitable substituents being: halogen, cyano, nitro, in each case straight-chain or branched alkyl, alkoxy, alkylthio or alkoxycarbonyl, having in each case 1 - 4 carbon atoms, in each case straight-chain or branched halogenoalkyl, halogenoalkoxy or halogenoalkylthio, having in each case 1 to 4 carbon atoms and 1 to 9 identical or different halogen atoms and also bivalent dioxyalkylene having 1 or 2 carbon atoms, optionally substituted by fluorine,

characterised in that α-cyanoacrylic acid derivatives of the formula (II)

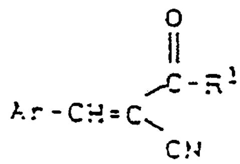

(II)

in which

Ar has the abovementioned meaning and

$R^1$ represents amino or alkoxy having 1 to 4 carbon atoms,

are reacted with isocyanoacetic acid esters of the formula (III)

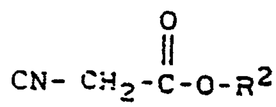

(III)

in which

$R^2$ represents alkyl having 1 to 4 carbon atoms,

in the presence of sodium hydride, sodium amide, sodium hydroxide or potassium hydroxide and if appropriate in the presence of a diluent at temperatures between 0 - 50 °C, 1.0 to 2.0 mol of isocyanoacetic acid ester of the formula (III) and 1.0 to 6.0 mol of the abovementioned bases being employed per mole of $\alpha$-cyanoacrylic acid derivative of the formula (II), and the $\Delta^2$-pyrroline-2-carboxylic acid derivatives thus obtainable, of the formula (IVa)

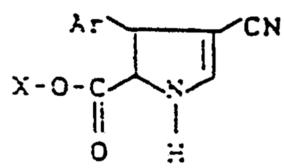

(IVa)

in which

X represents hydrogen or an equivalent of an inorganic or organic cation and

Ar has the abovementioned meaning,

are oxidatively decarboxylated in a 2nd step, if appropriate in the presence of an alkali metal hydroxide, alkali metal carbonate or of a tertiary amine and in the presence of a copper or iron(III) salt and if appropriate in the presence of a diluent at temperatures between 20 and 120 °C, 0.01 to 3.0 mol of copper salt or iron(III) salt and if appropriate 0.1 to 3.0 mol of one of the bases listed above being employed per mole of $\Delta^2$-pyrroline-2-carboxylic acid derivative of the formula (IVa).

**2.** Process according to Claim 1, characterised in that the 1st and 2nd steps are carried out without isolation of the intermediates of the formula (IVb)

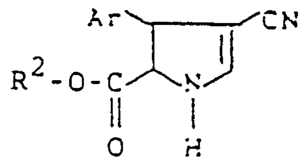

(IV b)

in which

$R^2$ and Ar have the meanings given in Claim 1.

3. Process according to Claim 1, characterised in that the 1st and 2nd steps are carried out in the presence of organic solvents.

4. Process according to Claim 1, characterised in that the 1st and 2nd steps are carried out in the presence of the bases listed in Claim 1.

5. Process according to Claim 1, characterised in that the 2nd step is carried out in the presence of a copper or iron(III) salt.

6. Pyrrole-2-carboxylic acid derivatives of the general formula (IV), according to Claim 1

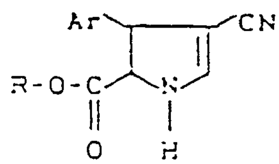

(IV)

in which

R represents hydrogen, alkyl having 1 to 4 carbon atoms or an equivalent of an inorganic or organic cation and Ar has the meaning indicated in Claim 1.

7. Process for the preparation of $\Delta^2$-pyrroline-2-carboxylic acid derivatives of the general formula (IV) according to Claim 1

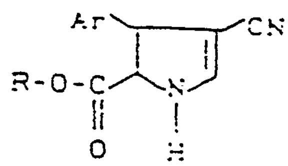

(IV)

in which

R represents hydrogen, alkyl having 1 to 4 carbon atoms or an equivalent of an inorganic or organic cation and Ar has the meaning indicated in Claim 1,

characterised in that α-cyanoacrylic acid derivatives of the formula (II)

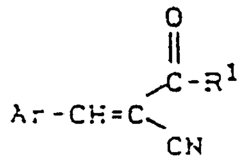

(II)

in which

Ar has the abovementioned meaning and

$R^1$ represents amino or alkoxy having 1 to 4 carbon atoms,

are reacted with isocyanoacetic acid esters of the formula (III)

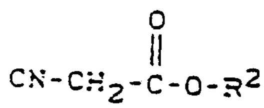

(III)

in which

$R^2$ represents alkyl having 1 to 4 carbon atoms,

in an aprotic solvent at temperatures between -20° C and 30° C in the presence of quasi-molar amounts of hydroxide base or anhydrous organic bases, and if appropriate the compounds obtained are hydrolysed

## Revendications

1. Procédé de préparation de 3-cyano-4-aryl-pyrroles de formule générale (I),

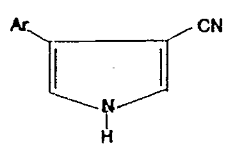

(I)

dans laquelle
Ar désigne respectivement un radical pyridyle, furyle ou thiényle éventuellement substitué une ou plusieurs fois, de manière identique ou différente par un halogène et un radical alkyle à chaîne droite ou ramifiée ayant de 1 à 4 atomes de carbone ou désigne un radical phényle éventuellement substitué une ou plusieurs fois, de manière identique ou différente, les substituants envisagés étant : un halogène, un cyano, un nitro, un radical alkyle, alkoxy, alkylthio ou alkoxycarbonyle à chaîne droite ou ramifiée, avec respectivement 1 à 4 atomes de carbone, un radical halogénalkyle, halogénalkoxy ou halogénalkylthio à chaîne droite ou ramifiée avec respectivement 1 à 4 atomes de carbone et 1 à 9 atomes d'halogène identiques ou différents ainsi qu'un radical dioxyalkylène deux fois lié, éventuellement substitué par du fluor et ayant 1 ou 2 atomes de carbone,

caractérisé en ce que des dérivés d'acide α-cyanoacrylique de formule (II)

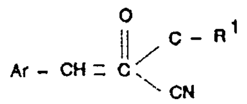

(II)

dans laquelle

Ar a la signification susmentionnée et
$R^1$ désigne un radical amino ou alkoxy avec 1 à 4 atomes de carbone

sont amenés à réagir avec des esters d'acide isocyanoacétique de formule (III),

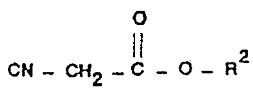

(III)

dans laquelle

$R^2$ désigne un radical alkyle avec 1 à 4 atomes de carbone,

en présence d'hydrure de sodium, d'amidure de sodium, d'hydroxyde de sodium ou d'hydroxyde de potassium et, éventuellement, en présence d'un diluant à des températures de l'ordre de 0 à 50 °C, 1,0 à 2,0 moles d'ester d'acide isocyanoacétique de formule (III) et 1,0 à 6,0 moles des bases susmentionnées étant utilisées par mole de dérivé d'acide α-cyanoacrylique et les dérivés d'acide$\Delta^2$-pyrrolino-2-carboxylique ainsi obtenus de formule (IVa),

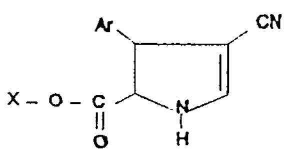

(IVa)

dans laquelle

X désigne l'hydrogène ou un équivalent d'un cation inorganique ou organique et
Ar a la signification susmentionnée,

sont décarboxylés par oxydation au cours d'une 2ème étape, éventuellement, en présence d'un hydroxyde alcalin, d'un carbonate de métal alcalin ou d'une amine tertiaire et en présence d'un sel de cuivre ou sel ferrique ainsi qu'éventuellement, en présence d'un diluant à des températures de l'ordre de 20 à 120 °C, 0,01 à 3,0 moles de sel de cuivre ou de sel ferrique et, éventuellement, 0,1 à 3,0 moles d'une des bases susmentionnées étant utilisées par mole de dérivé d'acide$\Delta^2$-pyrrolino-2-carboxylique de formule (IVa).

2. Procédé selon la revendication 1, caractérisé en ce que la lère et la 2ème étapes sont exécutées sans isoler les produits intermédiaires de formule (IVb)

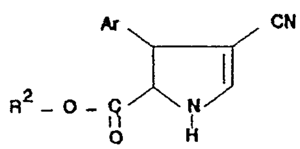

(IVb)

dans laquelle

$R^2$ et Ar ont les significations données dans la revendication 1.

3. Procédé selon la revendication 1, caractérisé en ce que la lère et la 2ème étapes sont exécutées en présence de solvants organiques.

4. Procédé selon la revendication 1, caractérisé en ce que la lère et la 2ème étapes sont exécutées en présence des bases présentées dans la revendication 1.

5. Procédé selon la revendication 1, caractérisé en ce que la 2ème étape est exécutée en présence d'un sel de cuivre ou d'un sel ferrique.

6. Dérivés d'acide pyrrolo-2-carboxylique de formule générale (IV), conformément à la revendication 1

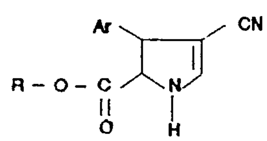

(IV)

dans laquelle

R désigne l'hydrogène, un radical alkyle avec 1 à 4 atomes de carbone ou un équivalent d'un cation inorganique ou organique et Ar a la signification donnée dans la revendication 1.

7. Procédé de préparation de dérivés d'acide$\Delta^2$-pyrrolino-2-carboxylique de formule générale (IV), conformément à la revendication 1

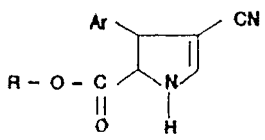

(IV)

dans laquelle

R désigne l'hydrogène, un radical alkyle avec 1 à 4 atomes de carbone ou un équivalent d'un cation inorganique ou organique et Ar a la signification donnée dans la revendication 1,

caractérisé en ce que les dérivés d'acide α-cyanoacrylique de formule (II),

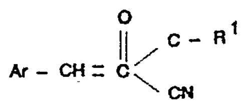

(II)

dans laquelle

Ar a la signification susmentionnée et
$R^1$ désigne un radical amino ou alkoxy avec 1 à 4 atomes de carbone

sont amenés à réagir avec des esters d'acide isocyanoacétique de formule (III),

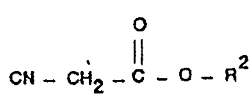

(III)

dans laquelle

$R^2$ désigne un radical alkyle avec 1 à 4 atomes de carbone,

dans un solvant aprotique, à des températures de l'ordre de - 20° C à 30° C, en présence de quantités quasi molaires de bases d'hydroxyde ou de bases organiques anhydres et que les composés obtenus sont éventuellement saponifiés.